# FASCICULE DE BREVET EUROPEEN

(11) **EP 2 137 133 B1**
(45) Date de publication et mention de la délivrance du brevet: **07.09.2011**
(21) Numéro de dépôt: 08788095.1
(22) Date de dépôt: 01.04.2008
(51) Int. Cl.: C07C 227/04, C07C 227/08, C07C 229/08, C07C 68/00, C07C 69/96

(54) **PROCEDE DE CO-PRODUCTION DE CARBONATES NON CYCLIQUES ET D'AMINO-ACIDES**
VERFAHREN ZUR GLEICHZEITIGEN HERSTELLUNG VON NICHTZYKLISCHEN CARBONATEN UND AMINOSÄUREN
METHOD FOR THE CO-PRODUCTION OF NON-CYCLIC CARBONATES AND AMINO ACIDS

(30) Priorité: 06.04.2007 FR 0754347
(43) Date de publication de la demande: 30.12.2009
(73) Titulaire: ARKEMA FRANCE, 92700 Colombes (FR)
(72) Inventeur: DUBOIS, Jean-Luc, F-69390 Millery (FR)
(74) Mandataire: Sauvageot, Olivier Roger
(86) Numéro de dépôt international: PCT/FR2008/050569
(87) Numéro de publication internationale: WO 2008/139080

(56) Documents cités:
- WO-A-03/093215
- US-A- 5 902 894
- WANG ET AL: "Synthesis of dimethyl carbonate from urea and methanol over solid base catalysts" CATALYSIS COMMUNICATIONS, ELSEVIER SCIENCE, AMSTERDAM, NL, vol. 7, no. 1, janvier 2006 (2006-01), pages 6-10, XP005199681 ISSN: 1566-7367 cité dans la demande

## Description

L'invention vise un procédé conjugué de synthèse d'une part d'acides ω-amino-alcanoïques ou d'esters de ces acides et d'autre part de carbonates de mono-alcools, à partir d'acides gras issus d'huiles naturelles.

L'évolution actuelle en matière d'environnement conduit dans les domaines de l'énergie et de la chimie à privilégier l'exploitation des matières premières naturelles provenant d'une source renouvelable. Ces contraintes environnementales imposent également d'éviter le transport et le stockage de matières premières dangereuses.

Un exemple de procédé industriel utilisant un acide gras comme matière première est celui de la fabrication à partir de l'acide ricinoléique extrait de l'huile de ricin, de l'acide amino-11-undécanoïque, qui est à la base de la synthèse du Rilsan^{®} 11. Ce procédé est décrit dans l'ouvrage « Les Procédés de Pétrochimie » de A. Chauvel et al. paru aux Editions TECHNIP (1986). L'acide amino-11-undécanoïque est obtenu en plusieurs étapes. La première consiste en une méthanolyse de l'huile de ricin en milieu basique produisant le ricinoléate de méthyle qui est ensuite soumis à une pyrolyse pour obtenir, d'une part l'heptanaldéhyde et, d'autre part l'undécylénate de méthyle. Ce dernier est passé en forme acide par hydrolyse. Ensuite l'acide formé est soumis à une hydrobromuration pour donner l'acide ω-bromé d'où l'on passe par amination à l'acide amino-11-undécanoïque.

On ne trouve dans la littérature pas d'autres documents décrivant la synthèse de composés du type aminoacides à partir d'acides gras d'origine naturelle. La demanderesse a récemment déposé une demande de brevet français sous le numéro FR 07.53285, publiée sous le numéro FR 2 912 741 A, décrivant des procédés de synthèse de ces ω-aminoacides à partir d'acides/esters gras naturels en mettant en oeuvre une réaction de métathèse croisée notamment avec l'acrylonitrile.

Le procédé de synthèse de l'acide amino-11-undécanoïque, opéré de manière industrielle depuis plusieurs décennies donne globalement satisfaction. Cependant, il présente un certain nombre d'inconvénients. Le premier inconvénient est que sa mise en oeuvre est en pratique asservie à l'accès à une <Le document WO 03/093215 A décrit la préparation d'acides ω-amino-alcanoïques à partir d'esters insaturés d'acides gras issus d'huiles naturelles. Ce procédé comprend une réaction de métathèse avec une oléfine, de préférence l'éthylène, conduisant à un ester ω-insaturé à chaine réduite. Ce composé intermédiaire est ensuite traité par hydroformylation et amination réductive pour conduire à l'acide ω-amino-alcanoïque.> matière première spécifique, l'huile de ricin. Le deuxième inconvénient est lié aux réactifs utilisés, l'ammoniac et le brome en particulier, qui nécessitent des précautions de stockage et d'utilisation coûteuses.

Le procédé industriel produit lors de sa première étape du glycérol ainsi que lors des étapes ultérieures de nombreux sous-produits qu'il faut valoriser séparément : de l'heptanaldéhyde, de l'estérol (mélange des esters d'acide gras non craqués). Il produit aussi du méthanol lors de l'étape d'hydrolyse. Ce méthanol est généralement traité pour être recyclé pour effectuer l'étape de méthanolyse.

Il existe plusieurs voies connues de synthèse du carbonate de mono-alcools tels que le diméthylcarbonate de formule CH₃O-CO-OCH₃ (DMC) ou le diéthylcarbonate (C₂H₅O-CO-OC₂H₅).

Les procédés industriels les plus anciens utilisent le phosgène comme matière première qui réagit avec l'alcool en milieu concentré de soude et cela en deux étapes successives, formation tout d'abord d'un chlorocarbonate puis du carbonate neutre. Ces procédés présentent des problèmes de sécurité en raison de la haute toxicité du phosgène ainsi que des déchets formés au cours du processus.

Les industriels se sont tournés alors vers des procédés de carbonylation oxydante ou des procédés de transestérification du carbonate d'éthylène.

Les procédés de carbonylation oxydante avec le couple oxydant CO + O₂ ont été développés soit en phase liquide, soit en phase gazeuse. Pour la phase liquide, on peut citer le procédé Enichem, brevets EP 534 545 et US 5,210,269, conduit en deux étapes en présence de chlorure de cuivre comme catalyseur. Pour la phase gazeuse, on peut citer le procédé UBE, brevets US 5,885,917, qui utilise NO comme agent actif avec un catalyseur au palladium, procédé comportant deux étapes avec le nitrite de méthyle comme produit intermédiaire.

Les dialkylcarbonates peuvent également être préparés par transestérification des carbonates d'alkylène avec l'alcool correspondant. De nombreuses sociétés ont travaillé sur ce type de réaction. On peut citer les brevets US 3,642,858 et US 3,803,201 de la société Dow qui décrivent un procédé de transestérification de carbonates d'alkylène avec des composés hydroxyles non tertiaires en présence de catalyseurs alcalins. La société Bayer a développé un procédé similaire en utilisant un catalyseur au Thallium (brevet US 4,307,032). Texaco propose pour sa part des catalyseurs solubles à base de sels de zirconium, titane et/ou étain (brevet US 4,661,609) ou à base de composés trivalents soufre ou sélénium (brevet US 4,734,518). Enfin plus récemment la société Nippon Shokubai décrit l'utilisation de catalyseurs supportés d'oxydes de terres rares (US 5,430,170).

Dans la demande WO 2004/091778, Mitsubishi décrit une synthèse de dialkylcarbonates utilisant du CO₂ supercritique et un catalyseur solide acide hétérogène, pour faire réagir le CO₂ sur l'acétone diméthylacétal.

Parmi les méthodes de synthèse des dialkylcarbonates on relève les réactions de l'urée sur les mono-alcools.

Les réactions de carbonatation utilisant de l'urée ont déjà été décrites pour des mono-alcools par exemple par « Shaikh et Sivaram, Organic Carbonates, Chem rev. 1986, 96 p951-576 », « Ball et al., Synthesis of carbonates and polycarbonates by reaction with urea with hydroxy compounds, Mol. Chem. 1984, vol.1, p95-108 ».

M. Wang, et al. dans « Catalysis Communication 7 (2006) 6-10 » décrivent la synthèse de diméthylcarbonate à partir de méthanol et d'urée sur des solides basiques. Cette réaction comporte deux étapes avec la formation intermédiaire du méthylcarbamate lui-même transformé en diméthylcarbonate. Malheureusement si la première étape permet l'obtention de bons résultats la seconde est beaucoup plus délicate avec des réactions secondaires, et dans ce cas les sélectivités de la réaction globale restent faibles.

Dans un brevet US 5,902,894, Catalytic Distillation Technologies décrit un procédé de synthèse du DMC utilisant des solvants organiques à haut point d'ébullition et donneurs d'électron ; par exemple le diméthyléther du triéthylèneglycol, associé à un catalyseur à l'étain permettait d'obtenir de bons rendements et de bonnes sélectivités en DMC en pratiquant une extraction par distillation en continu du DMC formé. De manière analogue, Exxon, dans la demande de brevet WO 95/17369, minimise la formation sous-produits par une distillation extractive du dialkylcarbonate.

Enfin, plus récemment, la demande de brevet chinois CN1431190 (Shanxi Institute) décrit un procédé de synthèse de DMC à partir de méthanol et d'urée, utilisant des catalyseurs à base d'étain et d'un co-catalyseur.

Dans le procédé de synthèse d'acides ω-amino-alcanoïques ou d'esters de ces acides, il est nécessaire d'importer et de stocker des quantités importantes d'ammoniac, ce qui représente outre le problème du transport, un risque majeur en cas de fuite, non seulement pour le personnel mais aussi pour le voisinage. Ce stockage impose par conséquent des contraintes importantes au site exploitant ce type de procédé.

Le problème à résoudre est de limiter ces risques environnementaux tout en maintenant les performances du procédé ou même mieux en les améliorant.

L'invention vise donc un procédé conjugué de production d'acides ω-amino-alcanoïques et de carbonates de mono-alcools à partir d'un ester d'acide gras mono insaturé issu d'huiles naturelles, dans lequel le même ester d'acide gras sert de matière première pour la synthèse d'acides ω-amino-alcanoïques d'une part et de carbonates de mono-alcools d'autre part. La synthèse des carbonates de mono-alcool est réalisée par action de l'urée sur le mono-alcool, l'ammoniac produit par cette réaction est utilisée comme réactif pour l'étape d'amination lors de la dernière étape de la synthèse des acides ω-amino-alcanoïques.

L'invention vise un procédé conjugué de production d'acides ω-amino-alcanoïques et de carbonates de mono-alcool (dialkylcarbonates de formule R₁O-CO-OR₁ dans laquelle R₁ comporte de 1 à 4 atomes de carbone) à partir d'un ester gras mono insaturé issu d'huiles naturelles comportant les zones suivantes :
I) zone de synthèse d'un acide ω-amino-alcanoïque comportant les étapes suivantes a) transformation tout d'abord de l'ester de l'acide gras, issu d'huiles d'origine naturelle, de formule CH₃-(CH₂)ₙ-CHR-CH₂-CH=CH-(CH₂)ₚ-COOR, dans laquelle R est soit H soit OH, R₁ un radical alkyle comprenant de 1 à 4 atomes de carbone, et n et p des indices compris entre 2 et 11, en un ester gras ω-insaturé réalisée soit par éthénolyse, soit par pyrolyse, suivie b) d'une hydrolyse de l'ester issu de l'étape a) transformant l'ester en acide correspondant et produisant simultanément l'alcool R₁OH qui est récupéré et adressé à la zone III, puis c) d'une hydrobromuration de l'acide ω-insaturé issu de l'étape b), puis d) d'une amination à l'ammoniac réalisée généralement avec de l'ammoniaque (solution aqueuse d'ammoniac) du composé résultant de l'hydrobromuration de l'étape c),
II) zone de synthèse d'un carbonate de mono-alcool par réaction de l'urée sur l'alcool R₁OH, la réaction dudit l'alcool avec l'urée produisant également de l'ammoniac,
III) zone de récupération et de purification de l'alcool R₁OH qui sert d'alimentation pour la zone II,
IV) zone de récupération de l'ammoniac issue de la zone III ainsi que celle issue de la zone Id où la réaction est réalisée avec un excès d'ammoniac, pour servir d'alimentation pour l'amination de l'étape d) de la zone I.

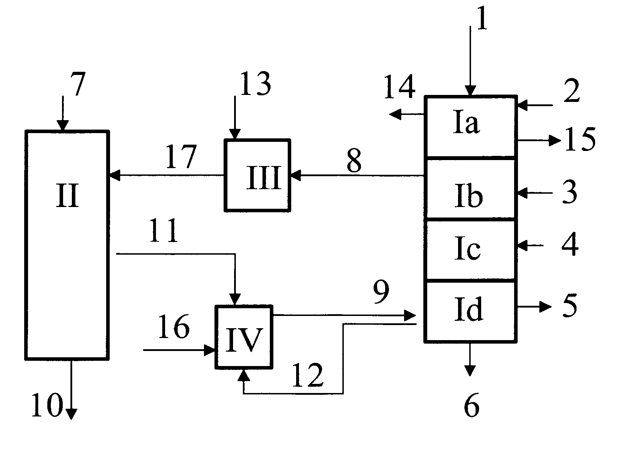

Le procédé de l'invention est décrit en référence au schéma simplifié annexé en figure 1 en prenant pour exemple la synthèse du DMC. Il va de soi qu'exactement le même processus peut être mis en oeuvre avec des esters d'acide gras tels que les esters éthylique, propylique, isopropylique ou butylique permettant la synthèse à côté de l'acide ω-amino-alcanoïque, de diéthylcarbonate, dipropylcarbonate, di(iso)propylcarbonate, diterbutylcarbonate ou di(iso)butylcarbonate. Dans le cas où l'ester à traiter n'est pas disponible sur le marché, il est naturellement possible de procéder au préalable à l'estérification de l'acide gras présent dans l'huile naturelle.

La zone I est alimentée par la ligne 1 en un ester mono insaturé d'acide gras, acide provenant d'une huile naturelle, de formule CH₃-(CH₂)ₙ-CHOR-CH₂-CH=CH-(CH₂)ₚ-COOR, dans laquelle R₁ est un radical méthyle, éthyle, propyle, butyle ou isopropyle, où il est transformé en un ester gras ω-insaturé de longueur de chaîne réduite par rapport à celle de l'acide gras de la charge initiale. Cette transformation est obtenue soit par pyrolyse, soit par éthénolyse. Dans le premier cas la chaleur nécessaire à la réaction de pyrolyse qui correspond au processus réactionnel suivant

CH₃-(CH₂)ₙ-CHOH-CH₂-CH=CH-(CH₂)ₚ-COOR₁ + Δ → CH₂=CH-(CH₂)ₚ₊₁-COOR₁ + CH₃-(CH₂)ₙ-CHO

est introduite par la ligne 2, sous forme de vapeur d'eau surchauffée, puis extraite par la ligne 14. Le co-produit de la réaction, par exemple l'heptanal dans le cas du ricinoléate d'alkyle, est extrait par la ligne 15.
Dans le second cas, on introduit dans la zone la par la ligne 2 de l'éthylène qui réagit avec l'ester insaturé, selon le processus illustré ci-après :

CH₃-(CH₂)ₙ-CH=CH-(CH₂)ₚ-COOR₁ + CH₂=CH₂ ⇔ CH₂=CH-(CH₂)ₚ-COOR₁ + CH₂=CH-(CH₂)ₙ-CH₃

pour former l'ester gras ω-insaturé d'une part, et une α-oléfine d'autre part. L'α-oléfine formée est extraite par la ligne 15.
L'ester gras ω-insaturé formé en zone la est ensuite transféré en zone Ib où il est soumis à une hydrolyse afin d'obtenir l'acide gras ω-insaturé correspondant. L'eau est introduite par la ligne 3 et le méthanol extrait par la ligne 8. Ce méthanol est adressé à la zone III pour servir après purification à l'alimentation de la zone II. L'acide gras ω-insaturé issu de la zone Ib est introduit dans la zone Ic où il est soumis à une hydrobromuration pour obtenir l'acide ω-bromo-alcanoïque selon le processus réactionnel suivant :

CH₂=CH-(CH₂)ₚ-COOH + HBr → BrCH₂-(CH₂)ₚ₊₁-COOH

L'acide bromhydrique ainsi que les composants nécessaires à la réaction sont introduits par la ligne 4.
L'acide ω-bromo-alcanoïque issu de la zone Ic est introduit dans la zone Id et est soumis à une amination par un excès d'ammoniac selon le processus réactionnel suivant :

BrCH₂-(CH₂)ₚ₊₁-COOH + 2 NH₃ → NH₂-(CH₂)ₚ₊₂-COOH + NH₄Br

L'ammoniac est introduit dans la zone Id par la ligne 9. L'acide ω-amino alcanoïque est extrait de la zone I par la ligne 6. L'acide bromhydrique sous forme de bromure d'ammonium formé est extrait par la ligne 5 et l'excès d'ammoniac renvoyé à la zone IV par la ligne 12.

L'alcool R₁OH issu de la zone Ib est adressé par la ligne 8 à la zone III. Cet alcool, après purification, sert d'alimentation par la ligne 17 de la zone II pour la réaction avec l'urée introduit par la ligne 7 pour former le carbonate de l'alcool R₁OH selon le processus réactionnel suivant :

2 R₁OH + NH₂-CO-NH₂ → R₁O-CO-OR₁ + 2 NH₃

Le carbonate de l'alcool R₁OH synthétisé est extrait de la zone II par la ligne 10 et l'ammoniac formé est adressée à la zone IV par la ligne 11.
La zone IV comporte outre les lignes d'alimentation 11 et 12, une ligne d'appoint 16 permettant notamment de fournir l'ammoniac nécessaire au démarrage de l'unité et à sa régulation en cas de variation de la production d'ammoniac au niveau de la zone II.

Le procédé est particulièrement bien adapté à la synthèse de l'acide amino-11-undécanoïque et de diméthylcarbonate à partir du ricinoléate de méthyle produit par méthanolyse de l'huile de ricin. Les différentes réactions seront les suivantes :
Zone la : CH₃-(CH₂)₅-CHOH-CH₂-CH=CH-(CH₂)₇-COOCH₃ + Δ → CH₂=CH-(CH₂)₈-COOCH₃ + CH₃-(CH₂)₅-CHO
Zone Ib : CH₂=CH-(CH₂)₈-COOCH₃ + H₂O → CH=CH-(CH₂)₈-COOH + CH₃OH
Zone Ic : CH₂=CH-(CH₂)₈-COOH + HBr → BrCH₂-(CH₂)₉-COOH
Zone Id : BrCH₂-(CH₂)₉-COOH + 2 NH₃ → NH₂- (CH₂)₁₀COOH + NH₄Br
Zone II : 2 CH₃OH + NH₂-CO-NH₂ → CH₃O-CO-OCH₃ + 2 NH₃

On pourra utiliser exactement le même procédé avec une réaction de pyrolyse dans la zone IIa pour la synthèse de l'acide amino-13-tridécanoïque en utilisant pour charge l'ester de l'acide lesquérolique répondant à la formule suivante :

CH₃-(CH₂)₅-CHOH-CH₂-CH=CH-(CH₂)₉-COOR₁

Pour la synthèse d'autres ω-aminoacides, on aura le plus souvent recours lors de l'étape la à une réaction de métathèse avec l'éthylène (ou éthénolyse).

Pour illustrer le mécanisme réactionnel en prenant pour exemple la synthèse de l'acide amino-7-heptanoïque, on utilisera comme matière première l'ester de l'acide pétrosélinique (acide cis-6-octadécènoïque) qui est soumis à une éthénolyse selon le processus suivant :

CH₃-(CH₂)₁o-CH=CH-(CH₂)₄-COOR, + CH₂=CH₂ ⇔ CH₂=CH-(CH₂)₄-COOR₁ + CH₂=CH-(CH₂)₁₀-CH₃

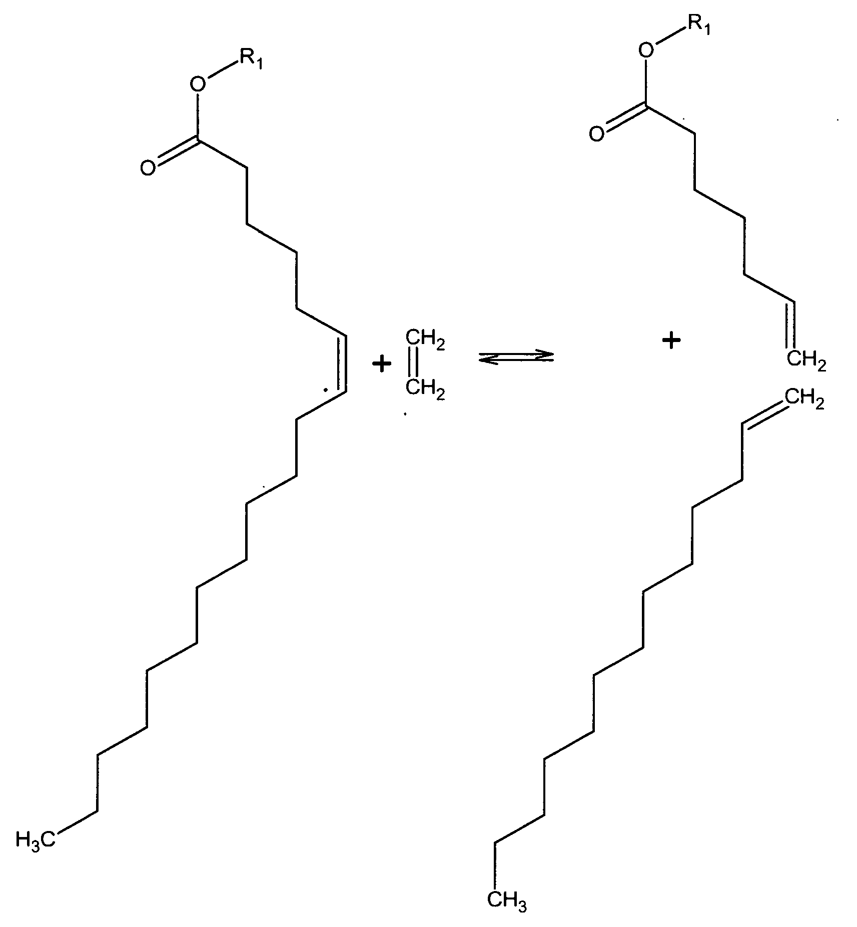

En utilisant le même processus, on pourra synthétiser divers ω-amine acides. On peut citer l'acide amino-6-hexanoïque en utilisant une charge contenant de l'acide lauroléique (acide cis-5-lauroléique). De même, on pourra synthétiser l'acide amino-10-décanoïque en utilisant une charge contenant des acides tels que les acides oléique (acide cis-9-octadécènoïque), palmitoléique (acide cis-9-hexadécènoïque), gadoléique (acide cis-9-eicosènoïque) et myristoléique (acide cis-9-tetradécènoïque). Pour la synthèse de l'acide amino-12-dodécanoïque, les charges utilisables sont celles contenant les acides vaccénique (acide cis-11-octadécènoïque), gondoïque (acide cis-11-eicosènoïque) et cétoléique (acide cis-11-docosènoïque). Enfin, pour la synthèse de l'acide amino-14-tétradécanoïque on pourra utiliser une charge contenant l'acide érucique (acide cis-13-docosènoïque).

Les conditions de mise en oeuvre des différentes étapes des zones I est II, sont connues de l'homme de l'art. On peut cependant préciser que :
- la pyrolyse de l'étape a) de la zone I est conduite à une température généralement comprise entre 400 et 600 °C.
- pour la métathèse de l'étape a) de la zone I, tout catalyseur de métathèse actif et sélectif pourra être utilisé. On utilisera cependant de préférence des catalyseurs à base de ruthénium.
- la réaction d'hydrolyse de l'étape b) de la zone II est réalisée par exemple à la température ambiante.
- l'hydrobromuration de l'étape c) de la zone I est réalisée à basse température en présence d'un solvant organique (par exemple toluène).
- l'amination de l'étape d) de la zone I est réalisée à température ambiante en présence d'ammoniac en excès dans une solution aqueuse.
- la conversion de l'alcool R₁OH en dialkylcarbonate réalisée au niveau de la zone II est comme cela a été indiqué précédemment une réaction connue depuis plusieurs d'années. Pour la conduite du procédé, la solution retenue est la réaction généralement nommée transestérification de l'alcool R₁OH par l'urée. Cette réaction est conduite en présence de catalyseurs connus en soi, par exemple ceux décrits dans les brevets US 6,495,703, oxyde de zinc, ou EP 0 955 298, sulfates métalliques ou organométalliques, et de préférence des catalyseurs à base d'étain tels que ceux décrits dans les brevets US 5,902,894, WO Application 95/17369 ou CN 1431190.

Les zones III et IV sont des zones de stockage et de purification de l'alcool R₁OH d'une part, et de l'ammoniac d'autre part, qui sont réalisées selon des techniques bien connues de l'homme de l'art. L'alcool est par exemple séparé de l'eau par distillation et par séchage sur des tamis moléculaires. L'ammoniac est utilisée sous forme de solution aqueuse.

On peut observer que le procédé peut être considéré comme quasi autonome en bilan matière ce qui est exceptionnel. En effet, l'étape Ib produit une quantité de mono-alcool qui est transférée dans la zone II pour réagir avec l'urée dans le rapport de 2 moles d'alcool pour une mole d'urée. Cette réaction de synthèse du dialkylcarbonate produit elle-même deux moles d'ammoniac, quantité juste nécessaire à l'amination de l'étape Id de la zone I. Outre l'économie entraînée, la limitation du stockage d'ammoniac sur le site est bénéfique sur le plan environnemental.

## Revendications

1. Procédé conjugué de production d'acides ω-amino-alcanoïques et de carbonates de mono-alcool, dialkylcarbonates de formule R₁O-CO-OR₁ dans laquelle R₁ comporte de 1 à 4 atomes de carbone, à partir d'un ester mono insaturé d'un acide gras issu d'huiles naturelles comportant les zones réactionnelles suivantes :
I) zone de synthèse d'un acide ω-amino-alcanoïque comportant les étapes suivantes a) transformation tout d'abord dans la zone la de l'ester de l'acide gras de formule CH₃-(CH2)ₙ-CHR-CH₂-CH=CH-(CH₂)ₚ-COOR₁ introduit par la ligne 1 dans laquelle R est soit H soit OH, R₁ un radical alkyle comprenant de 1 à 4 atomes de carbone, et n et p des indices compris entre 2 et 11, en un ester gras ω-insaturé soit par éthénolyse, soit par pyrolyse, suivie b) dans la zone Ib d'une hydrolyse de l'ester issu de l'étape a) pour former l'acide correspondant avec simultanément formation de l'alcool R₁OH qui est récupéré et adressé par la ligne 8 à la zone III, puis c) d'une hydrobromuration dans la zone Ic de l'acide ω-insaturé issu de l'étape b), puis d) d'une amination à l'ammoniac introduite par la ligne 9 du composé résultant de l'hydrobromuration de l'étape c), l'acide ω-amino-alcanoïque étant extrait par la ligne 6 et l'ammoniac excédentaire recyclée en zone IV par la ligne 12,
II) zone de synthèse d'un carbonate de mono-alcool R₁OH, dialkylcarbonate de formule R₁O-CO-OR₁, par réaction de l'urée introduit par la ligne 7 avec R₁OH introduit par la ligne 17, la réaction de R₁OH avec l'urée produisant également de l'ammoniac qui est adressée par la ligne 11 à la zone IV, le carbonate produit étant extrait par la ligne 10,
III) zone de récupération et de purification de l'alcool R₁OH amené de la zone Ib par la ligne 8, pour servir par la ligne 17 d'alimentation de la zone II pour la synthèse des dialkylcarbonates, carbonates de mono-alcool R₁OH par réaction avec l'urée, une alimentation d'appoint en alcool R₁OH étant effectuée par la ligne 13,
IV) zone de récupération et de purification de l'ammoniac issue par la ligne 11 de la zone II ainsi que celle issue par la ligne 12 de la zone Id où la réaction est réalisée avec un excès d'ammoniac, pour servir d'alimentation par la ligne 9 pour l'amination de l'étape d) de la zone I, une alimentation d'appoint en ammoniac de la zone IV étant effectuée par la ligne 16.

2. Procédé selon la revendication 1 **caractérisé en ce que** l'ester de la charge répond à la formule CH₃-(CH₂)₅-CHOH-CH₂-CH=CH-(CH₂)₇-COOCH₃, qui est soumis lors de l'étape la à une pyrolyse pour former à la sortie des étapes de la zone I l'acide amino-11-undécanoïque, et le diméthylcarbonate à la sortie de la zone II.

## Claims

1. Combined process for the production of ω-aminoalkanoic acids and monoalcohol carbonates which are dialkyl carbonates of formula R₁O-CO-OR₁ in which R₁ comprises from 1 to 4 carbon atoms, from a monounsaturated ester of a fatty acid resulting from natural oils, comprising the following reaction regions:
I) region of synthesis of an ω-aminoalkanoic acid comprising the following stages a) first of all conversion, in the region Ia, of the ester of the fatty acid of formula CH₃- (CH₂)ₙ-CHR-CH₂-CH=CH-(CH₂)ₚ-COOR₁, introduced via the line 1, in which R is either H or OH, R₁ is an alkyl radical comprising from 1 to 4 carbon atoms and n and p are indices between 2 and 11, to an ω-unsaturated fatty ester, either by ethenolysis or by pyrolysis, followed b), in the region Ib, by hydrolysis of the ester resulting from stage a), in order to form the corresponding acid with simultaneously formation of the alcohol R₁OH, which is recovered and sent via the line 8 to the region III, then c) by hydrobromination, in the region Ic, of the ω-unsaturated acid resulting from stage b) and then d) by amination with ammonia, introduced via the line 9, of the compound resulting from the hydrobromination of stage c), the ω-aminoalkanoic acid being extracted via the line 6 and the excess ammonia being recycled to the region IV via the line 12,
II) region of synthesis of a monoalcohol R₁OH carbonate, which is a dialkyl carbonate of formula R₁O-CO-OR₁, by reaction of urea, introduced via the line 7, with R₁OH, introduced via the line 17, the reaction of R₁OH with urea also producing ammonia, which is sent via the line 11 to the region IV, the carbonate produced being extracted via the line 10,
III) region of recovery and purification of the alcohol R₁OH conveyed from the region Ib via the line 8, in order to act, via the line 17, as feed of the region II for the synthesis of the dialkyl carbonates, which are monoalcohol R₁OH carbonates, by reaction with urea, a make-up feed of alcohol R₁OH being supplied via the line 13,
IV) region of recovery and purification of the ammonia resulting, via the line 11, from the region II and that resulting, via the line 12, from the region Id, where the reaction is carried out with an excess of ammonia, in order to act as feed, via the line 9, for the amination of stage d) of the region I, a make-up feed of ammonia for the region IV being supplied via the line 16.

2. Process according to Claim 1, **characterized in that** the ester of the feedstock corresponds to the formula CH₃-(CH₂)₅-CHOH-CH₂-CH=CH-(CH₂)₇-COOCH₃, which is subjected, during stage Ia, to pyrolysis, in order to form, at the outlet of the stages of the region I, 11-aminoundecanoic acid and, at the outlet of the region II, dimethyl carbonate.

## Patentansprüche

1. Konjugiertes Verfahren zur Herstellung von ω-Aminoalkansäuren und Monoalkoholcarbonaten, Dialkylcarbonaten der Formel R₁O-CO-OR₁, in der R₁ 1 bis 4 Kohlenstoffatome umfasst, aus einem ungesättigten Monoester einer Fettsäure, die aus natürlichen Ölen hervorgeht, wobei das Verfahren die folgenden Reaktionszonen umfasst:
I) Zone zur Synthese einer ω-Aminoalkansäure, die die folgenden Stufen umfasst: a) zunächst Umwandlung des über die Leitung 1 eingebrachten Esters der Fettsäure der Formel CH₃-(CH₂)ₙ-CHR-CH₂-CH=CH-(CH₂)ₚ-COOR₁, in der R entweder H oder OH ist, R₁ ein Alkylrest ist, der 1 bis 4 Kohlenstoffatome umfasst, und n und p Zahlen zwischen 2 und 11 sind, in der Zone Ia in einen ωungesättigten Fettsäureester entweder durch Ethenolyse oder durch Pyrolyse, gefolgt von b) einer Hydrolyse des aus der Stufe a) hervorgegangenen Esters in der Zone Ib unter Ausbildung der entsprechenden Säure bei gleichzeitiger Bildung des Alkohols R₁OH, der gewonnen und über die Leitung 8 in die Zone III überführt wird, dann c) eine Hydrobromierung der aus der Stufe b) hervorgegangenen ω-ungesättigten Säure in der Zone Ic, dann d) eine Aminierung der aus der Hydrobromierung der Stufe c) resultierenden Verbindung mit dem über die Leitung 9 eingebrachten Ammoniak, wobei die ω-Aminoalkansäure über die Leitung 6 extrahiert wird und der überschüssige Ammoniak in Zone IV über die Leitung 12 rückgeführt wird,
II) Zone zur Synthese eines Carbonats des Monoalkohols R₁OH, eines Dialkylcarbonats der Formel R₁O-CO-OR₁, durch Reaktion des über die Leitung 7 eingebrachten Harnstoffs mit über die Leitung 17 eingebrachtem R₁OH, wobei die Reaktion von R₁OH mit dem Harnstoff ebenfalls Ammoniak produziert, der über die Leitung 11 in die Zone IV überführt wird, wobei das hergestellte Carbonat über die Leitung 10 extrahiert wird,
III) Zone zur Gewinnung und Reinigung des Alkohols R₁OH, der über die Leitung 8 in die Zone Ib geleitet wird, um über die Leitung 17 der Speisung der Zone II für die Synthese von Dialkylcarbonaten, Carbonaten des Monoalkohols R₁OH, durch Reaktion mit dem Harnstoff zu dienen, wobei eine Zusatzspeisung mit Alkohol R₁OH über die Leitung 13 bewirkt wird,
IV) Zone zur Gewinnung und Reinigung des Ammoniaks, der über die Leitung 11 von der Zone II stammt, sowie des Ammoniaks, der über die Leitung 12 von der Zone Id stammt, wo die Reaktion mit einem Ammoniaküberschuss durchgeführt wird, um der Speisung der Zone I über die Leitung 9 für die Aminierung der Stufe d) zu dienen, wobei eine Zusatzspeisung der Zone IV mit Ammoniak über die Leitung 16 bewirkt wird.

2. Verfahren nach Anspruch 1, **dadurch gekennzeichnet, dass** der Ester der Beschickung der Formel CH₃-(CH₂)₅-CHOH-CH₂-CH=CH-(CH₂)₇-COOCH₃ entspricht, der in der Stufe Ia einer Pyrolyse unterzogen wird, um am Ausgang der Stufen der Zone I Amino-11-undecansäure und am Ausgang der Zone II Dimethylcarbonat zu bilden.
